# EUROPÄISCHE PATENTSCHRIFT

(11) **EP 0 530 688 B1**
(45) Veröffentlichungstag und Bekanntmachung des Hinweises auf die Patenterteilung: **29.11.1995**
(21) Anmeldenummer: 92114687.4
(22) Anmeldetag: 28.08.1992
(51) Int. Cl.: A61M 1/36, B01D 43/00

(54) **Verfahren und Vorrichtung zum Trennen von Blut in seine Bestandteile**
Method and device for the separation of blood components
Procédé et appareil pour la séparation des composants du sang

(30) Priorität: 06.09.1991 DE 4129516
(43) Veröffentlichungstag der Anmeldung: 10.03.1993
(73) Patentinhaber: Fresenius AG, 61350 Bad Homburg (DE)
(72) Erfinder: Biesel, Wolfgang, Dr., W-6682 Otweiler (DE); Geibel, Johannes, W-6648 Wadern (DE); Brass, Henning, W-6650 Homburg (DE)
(74) Vertreter: Fuchs Mehler Weiss

(56) Entgegenhaltungen:
- FR-A- 2 406 449
- FR-A- 2 468 374

## Beschreibung

Die Erfindung betrifft ein Verfahren zum Trennen von Blut in seine Bestandteile durch Dichtezentrifugation, bei dem in einer ersten Trennstufe mit überwachter Phasengrenze das Blut grob in mindestens zwei Phasen separiert wird, von denen die erste Fraktion vorwiegend aus Blutzellen und die zweite Fraktion vorwiegend aus thrombozytenreichem Blutplasma besteht, und bei dem in einer zweiten Trennstufe eine der Fraktionen zwecks Gewinnung eines Konzentrates eines Blutbestandteiles weiter separiert wird. Die Erfindung betrifft ferner eine Vorrichtung zur Trennung von Blut in seine Bestandteile durch Dichtezentrifugation mit einer ersten Trennstufe, der Separationskammer, der eingangsseitig über eine Blutpumpe das zu trennende Blut zuführbar ist, in der das Blut grob in mindestens zwei Phasen separiert wird, von denen die erste Fraktion vorwiegend aus Blutzellen und die zweite Fraktion vorwiegend aus thrombozytenreichem Blutplasma besteht, und die eine Einrichtung zur Erfassung der Phasengrenze zwischen beiden Fraktionen aufweist mit einer zweiten Trennstufe, der Sammelkammer, der über eine Plasmapumpe die thrombozytenreiche Blutplasma -Fraktion zuführbar ist, und in der diese Fraktion zwecks Gewinnung eines Konzentrates eines Blutbestandteiles weiter separierbar ist, und mit einer Programmsteuerung zum Betrieb der Vorrichtung einschließlich der Einstellung der Pumpgeschwindigkeit der Plasmapumpe.

Vorzugsweise wird in der zweiten Trennstufe die thrombozytenreiche Plasmafraktion zwecks Gewinnung eines Thrombozytenkonzentrates weiter separiert.

Derartige Vorrichtungen sind z.B. durch den Zellseparator der Anmelderin Fresenius AS 104, der Firma Baxter CS 3000 oder der Firma Cobe Spectra bekannt geworden.

Zur Behandlung von Patienten bei bestimmten Krankheiten werden bestimmte Blutbestandteile benötigt, die dem Patienten zugeführt werden. So benötigt man beispielsweise zur Behandlung von thrombozytopenischen Patienten Thrombozytenkonzentrate.

Dazu wird das Blut eines Spenders, der an einen extrakorporalen Kreislauf angeschlossen ist, in einer Blutzentrifuge einer Dichtezentrifugation unterworfen und in seine Bestandteile getrennt. Bei dieser Blutseparation wird das Blut in einer ersten Stufe grob in zwei Phasen getrennt, nämlich in eine dunkelrote Zellkonzentratfraktion, die anfangs hauptsächlich aus Erythrozyten besteht, und in eine helle, gelbliche Fraktion, die hauptsächlich aus thrombozytenreichem Blutplasma besteht. Die Lage der Grenze zwischen beiden Phasen wird von einer Detektoreinrichtung überwacht und zum Beispiel mit Hilfe einer Plasmapumpe geregelt. Dabei spricht man von einer hohen Phasengrenze, wenn die Menge an Zellen die Menge an zellreichem Plasma überwiegt, und von einer niedrigen Phasengrenze im umgekehrten Fall. Bei dem bekannten Verfahren zur Gewinnung von Thrombozythenkonzentraten wird dabei in der üblichen Betriebsart die Lage der Phasengrenze nach Erreichen der gewünschten Lage während der Separationsdauer konstant gehalten.

Will man ein Thrombozytenkonzentrat gewinnen, wird das thrombozytenreiche Plasma in einer zweiten Stufe in thrombozytenarmes Plasma und das gewünschte Thrombozytenkonzentrat separiert. Das Thrombozytenkonzentrat dient zur Behandlung des Patienten, die restlichen Bestandteile des Blutes werden wieder vereinigt und dem Spender zugeführt.

Ein Standard-Thrombozytenkonzentrat, bestehend aus ungefähr 3 bis 4 x 10¹¹ Zellen, sollte mit einer möglichst geringen Separationsdauer, mit einer möglichst hohen Ausbeute und einer möglichst geringen Kontamination durch Leukozyten gewonnen werden. Leukozyten lösen nämlich in einem fremden Organismus, hier des Patienten, Abwehrmechanismen aus. Dies führt zur Immunisierung des Patienten, die die Wirksamkeit später verabreichter Präparate stark einschränken kann.

Die Lymphozyten, eine Unterfraktion der Leukozyten, die maßgebend für die Auslösung der Abwehrmechanismen sind, und die Thrombozyten können aufgrund der geringen Größenunterschiede durch Zentrifugation gemäß den bekannten Verfahren nicht sicher voneinander getrennt werden.

Nach dem Stand der Technik wird daher zur sicheren Vermeidung einer Immunisierung ein Thrombozytenkonzentrat zur Abtrennung der Leukozyten zusätzlich filtriert, bevor es einem Patienten zugeführt wird. Bei anderen Blutbestandteilen werden vergleichbare Maßnahmen durchgeführt.

Bei dem bekannten Verfahren ist daher ein weiterer Behandlungsschritt in einer weiteren Trenneinrichtung notwendig, wodurch mit Nachteil der Aufwand und die Kosten erhöht werden, auch hinsichtlich der Aufrechterhaltung der Sterilität.

Der Erfindung liegt die Aufgabe zugrunde, das eingangs beschriebene Verfahren sowie die eingangs bezeichnete Vorrichtung so auszugestalten, daß unmittelbar ein Blutbestandteil, insbesondere ein Thrombozytenkonzentrat gewonnen werden kann, welches extrem gering kontaminiert ist und daher nicht mehr gefiltert werden muß.

Die Lösung dieser Aufgabe gelingt gemäß der Erfindung bei dem Verfahren dadurch, daß in der ersten Trennstufe während des Trennvorgangs die Phasengrenze zwischen beiden Fraktionen alternierend zwischen mindestens zwei Stellungen verschoben wird.

Bei der Vorrichtung gelingt die Lösung dadurch, daß die Programmsteuerung 6 einen Steuerabschnitt für die Plasmapumpe 5 aufweist, der so ausgebildet ist, daß in der Separationskammer 1 während des Trennvorgangs die Phasengrenze zwischen den beiden Fraktionen alternierend zwischen mindestens zwei Stellungen verschiebbar ist.

Durch das Alternieren der Phasengrenzen in der ersten Trennstufe ist es möglich, im Falle der Gewinnung eines Thrombozytenkonzentrates gezielt nur Thrombozyten mit thrombozytenreichem Plasma in die zweite Trennstufe zu überführen und so die Kontamination durch Leukozyten fast vollständig zu eliminieren.

Es ist bekannt, bei Blutzellseparatoren der eingangs genannten Art eine Betriebsart mit konstant vorgegebener Trenngrenze vorzusehen, bei der die Plasmapumpe kurzzeitig auf eine erhöhte Geschwindigkeit gegenüber der Blutpumpe gefahren wird, um dann anschließend wieder auf die ursprüngliche Betriebsart zurückzufallen. Bei dieser "Spill-over"-Betriebsart tritt ein Überspülen der thrombozytenreichen Plasmafraktion (PRP-Fraktion) bis zum Übertreten der roten Blutzellen ein. Während dieser Zeit wird die Plasmapumpe des Blutzellseparators auf eine erhöhte Geschwindigkeit gefahren und stoppt z. B. bei Erkennung erhöhter Zelldichte oder nach Transfer eines voreingegebenen Volumens der zu sammelnden Fraktion. Bei diesem spill-over Verfahren bricht daher die Phasengrenze zusammen, um sich anschließend bei dem vorgegebenen konstanten Wert wieder einzustellen. Bei dem Verfahren der "alternierenden Trenngrenze" gemäß der Erfindung wird dagegen die Trenngrenze zwischen zwei konstanten Stellungen in zyklischen Abständen gewechselt. Nur während der Änderung der Trenngrenze wird die Plasmaflußrate geändert. In beiden konstanten Trenngrenzenpositionen wird die PRP-Fraktion in die zweite Stufe überführt und Blutzellen über einen 2. Ausgang aus der Separationskammer des Blutzellseparators gedrückt und zum Spender zurückgeführt. Das Verfahren ist ebenso bei der Gewinnung anderer Zellarten eines Gemisches von Zellen durch Separation anderer biologischer Flüssigkeiten anwendbar.

Die Erfindung wird im folgenden anhand der Zeichnungen und eines Ausführungsbeispieles näher beschrieben.

Als Ausführungsbeispiel wird die Gewinnung eines Thrombozytenkonzentrates beschrieben.

Es zeigen:
- Figur 1: in schematischer Darstellung die Verteilung der Phasen in einer Blut-Zentrifugiereinrichtung bei niedriger Phasengrenze,
- Figur 2: eine schematische Darstellung entsprechend Figur 1, jedoch bei hoher Phasengrenze,
- Figur 3: ein Diagramm, bei dem die Thrombozytenkonzentration auf der Ordinate gegen die Separationsdauer auf der Abszisse aufgetragen ist, bei konstanter Lage der Phasengrenze,
- Figur 4: ein Diagramm entsprechend Figur 3, jedoch mit alternierender Phasengrenze und
- Figur 5: ein Blockschaltbild eines Blutzellseparators (Blutzentrifuge).

Verfahren und Vorrichtungen zum Trennen von Blut in seine Bestandteile durch Dichtezentrifugation sind notorisch bekannt. Sie sind beispielsweise aus den US-Patenten 3 655 123, 4 056 224, 4 108 353 bekannt, auf die Bezug genommen wird. Einrichtungen zur Überwachung der Phasengrenze in solchen Trennvorrichtungen (Blutzentrifugen) sind beispielswiese aus der EP-Patentschrift 116 716 bekannt, auf deren Offenbarung Bezug genommen wird. Schematische Darstellungen in den Zeichnungen sind daher für das Verständnis der Erfindung ausreichend.

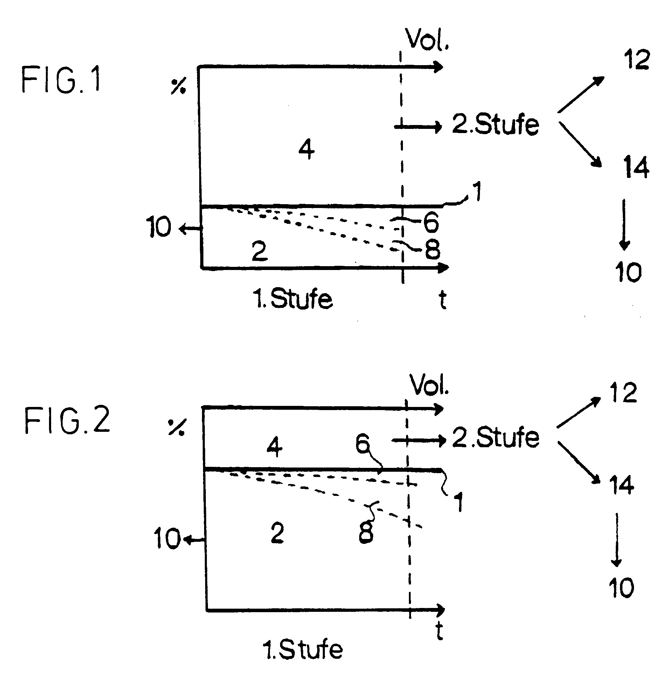

In Figur 1 ist schematisch abhängig von der Separationsdauer t die Verteilung der Phasen in einer Blutzentrifuge bei niedriger Trenngrenze 1 dargestellt. Im Verlauf der Separation bildet sich eine Schichtung der separierten Blutkomponenten aus. Auf Erythrozyten 2 aufgelagert und einer Schicht aus thrombozytenreichem Plasma 4 unterschichtet, bildet sich eine relativ breite Thrombozytenschicht 6 über einer Leukozytenschicht 8 aus. In der schematischen Darstellung ist ein fiktiver Idealzustand der Phasengrenze zwischen Thrombozyten und Leukozyten gezeigt. Es zeigt sich in der Praxis, daß die relativ breite Thrombozytenschicht eine Barriere gegenüber der Leukozytenschicht herzustellen vermag. Die Erythrozyten werden dem Spender 10 wieder zugeführt, und nur das thrombozytenreiche Plasma 4 wird in eine zweite Separationsstufe überführt. In dieser Stufe werden die Thrombozyten aufkonzentriert (Position 12) und das thrombozytenarme Plasma 14 nach Durchmischung mit den Erythrozyten 2 aus der ersten Stufe dem Spender 10 zurückgeführt.

Figur 2 stellt schematisch die Verteilung der separierten Bestandteile bei hoher Phasengrenze dar. Die Bezugszeichen sind wie in Figur 1 gewählt. Die Phasengrenze 1, auch Trenngrenze genannt, kann mit bekannten Mitteln verschoben und geregelt werden, zum Beispiel durch unterschiedlichen Abzug der Plasmafraktion mittels einer Plasmapumpe.

Im Gegensatz zur Separation bei niedriger Phasengrenze (Figur 1) bildet die Schicht der Thrombozyten 6 gegenüber der Schicht der weißen Blutzellen 8 (Leukozyten) eine kleinere Barriere aus. Wegen der höheren Strömungsgeschwindigkeit in der Plasmaschicht ist ein Sedimentieren der Blutzellen erschwert. Aus u.a. diesen Gründen kann wesentlich leichter eine Kontamination des thrombozytenreichen Plasmas 4 mit weißen Blutzellen 8 erfolgen.

Über die Dauer einer Separation ändert sich die Zusammensetzung des Zellkonzentrats innerhalb der ersten Separationsstufe. Die Tabelle 1 zeigt den Anteil der Blutfraktionen in Prozent in Abhängigkeit von der verarbeiteten Blutmenge bei mittlerer Lage der Trenngrenze, wobei die verwendeten Abkürzungen für die Blutfraktionen folgende Bedeutung haben: PRP = thrombozytenreiches Plasma; PLT = Thrombozyten; WBC = Leukozyten; RBC = Erythrozyten.

**Tabelle 1**

| Blutvolumen | PRP | PLT | WBC | RBC |
|---|---|---|---|---|
| 200 ml | 40 | 2 | 5 | 53 |
| 1.000 ml | 40 | 10 | 15 | 35 |
| 3.000 ml | 40 | 20 | 30 | 10 |

Bei konstanter Trenngrenzenposition kommt es gemäß Tabelle 1 mit zunehmendem Blutvolumen zu einer Anhäufung einer Zwischenphase in der Separationskammer, die hauptsächlich aus Thrombozyten und Leukozyten besteht. Plasma wird zur Regelung der Phasengrenze abgezogen und Erythrozyten aus der Separationskammer der Blutzentrifuge gedrückt.

Mit konstant niedriger Trenngrenzenposition in der ersten Separationsstufe ist erfahrungsgemäß eine Ausbeuteverlust an Thrombozyten in der zweiten Stufe verbunden. Andererseits wird hier ein Transfer von Leukozyten unterbunden und gewährleistet damit eine niedrige Leukozytenkontamination im Thrombozytenkonzentrat.

Bei konstant hoher Trenngrenzenposition in der ersten Separationsstufe steigt zwar die Anzahl an Thrombozyten in der zweiten Stufe, jedoch auch die Anzahl der Leukozyten und damit die Kontamination im Thrombozytenkonzentrat. Bei extrem hoher Phasengrenze nimmt die Kontamination sprunghaft zu, wie nachstehende Tabelle zeigt, in der die Effektivität und Leukozytenkontamination (WBC) von Thrombozytapheresen bei konstanter und alternierender Trenngrenzenposition dargestellt sind.

**Tabelle 2**

| Mittelwerte | Trenngrenze konstant | | Trenngrenze altern. niedrig/extrem hoch |
|---|---|---|---|
| | hoch | extrem hoch | |
| Effektivität % | 40,8 | 43,9 | 39,1 |
| WBC-Kontamination *10 E6 | 3,8 | 128,6 | 0,34 |

Durch erhöhte Strömungsgeschwindigkeit in der Plasmaschicht ist eine Ablagerung von Thrombozyten nicht möglich und es erfolgen Verwirbelungen und erhöhte Vermischung mit der Leukozytenschicht, so daß Leukozyten mit Thrombozyten in die zweite Stufe überführt werden können. Die Separationseffektivität in Bezug auf Thrombozyten erreicht zwar maximal hohe Werte, jedoch ist die Kontamination im Extremfall um das Hundertfache erhöht. Als Effektivität der Separation bezeichnet man hier das Verhältnis zwischen Ausbeute und Angebot der gewünschten Zellenart, ausgedrückt in Prozent. Das Angebot an einer Zellenart wird durch Ermitteln der mittleren Konzentration der Zellenart im angebotenen Blutvolumen bestimmt, die Ausbeute durch Ermitteln der Konzentration der Zellenart im Volumen des gewonnenen Konzentrats.

Das erfindungsgemäße Verfahren sieht vor, durch alternierenden Wechsel der Trenngrenzenposition zwischen mindestens zwei Stellungen während des Trennvorganges in der ersten Trennstufe eine Sammelzeit und eine Transferzeit zu schaffen. Während der Sammelzeit ist vorzugsweise eine niedrige bis hohe Trenngrenze eingestellt, so daß es zu einer Anhäufung von Thrombozyten in der Zwischenphase kommt. Ein Hauptteil der Thrombozyten wird kontinuierlich mit dem thrombozytenreichen Plasma in die zweite Stufe überführt. Die separierten Zellen werden aus der Trennstufe gedrückt.

In der Transferzeit wird die Phasengrenze extrem hochgefahren, so daß die in der ersten Stufe gesammelten Thrombozyten mit überspült werden. Ein gewisser Restgehalt der Blutplättchenschicht verbleibt in der Kammer und verhindert einen Übertritt der Leukozyten.

Zwar wird die Effektivität der Separation durch das Verfahren einer gewissen Thombozytenmenge etwas geringer, jedoch kann die Leukozytenkontamination weit unter das kritische immunologische Limit gedrückt werden und damit eine weitere Reinigungsstufe vermieden.

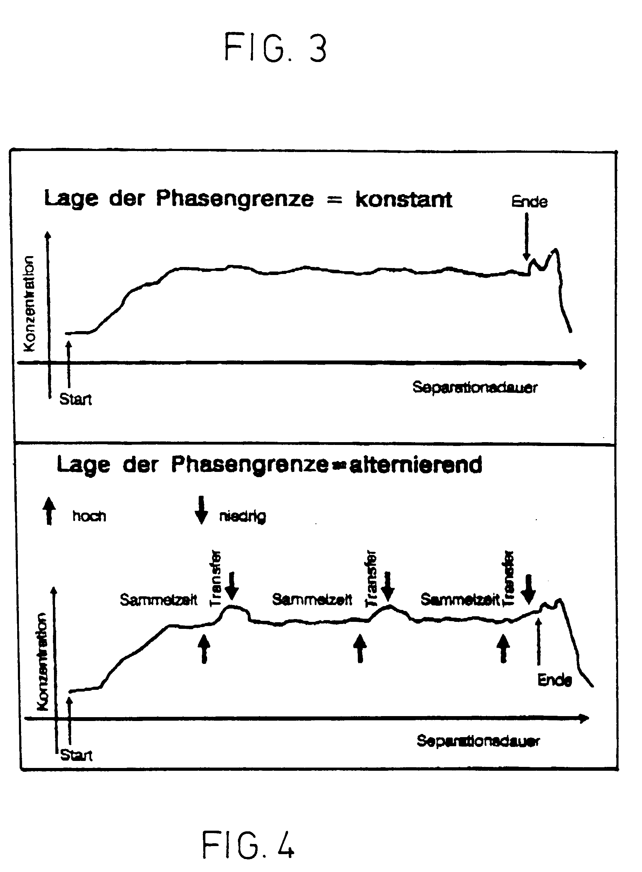

Diese Figur 4 zeigt ein Diagramm, bei dem die Thrombozytenkonzentration auf der Ordinate gegen die Separationsdauer auf der Abszisse aufgetragen ist, und zwar mit alternierenden Phasengrenzen gemäß der Erfindung. Die Figur 3 zeigt dabei ein entsprechendes Diagramm, jedoch mit konstanter Lage der Phasengrenze in der ersten Trennstufe gemäß dem Stand der Technik. Im Vergleich der beiden Diagramme erkennt man deutlich eine Thrombozytenzunahme im Bereich der Transferphase (Figur 4).

Bedingt durch die anfangs tiefliegende Trenngrenze, sowie durch die Veränderung der Lage der Trenngrenze und der damit veränderten Strömungsverhältnisse in der Kammer werden Leukozyten über den Zellenauslaß aus der Kammer entfernt.

Dadurch besteht während der Zeit der hochliegenden Trenngrenze keine Gefahr der Kontamination des thrombozytenreichen Plasmas und damit des daraus anschließend gewonnenen Thrombozytenkonzentrates. In dieser Phase ist jedoch der Gehalt an in die zweite Stufe überführten Thrombozyten wesentlich erhöht, wie in Fig. 4 dargestellt.

Die Zeit der hochliegenden Trenngrenze, in Abb. 4 Transferphase genannt, ist verhältnismäßig zur Zeit der niedrigliegenden Trenngrenze in Abb. 4 Sammelphase genannt, relativ kurz, da während dieser Zeit bereits eine neuerliche Ansammlung von u.a. Leukozyten beginnt.

Die Ermittlung sinnvoller Phasenlängen und Trenngrenzenpositionen kann empirisch erfolgen und wird dann dem jeweiligen Separationsprogramm der Trennvorrichtung vorgegeben.

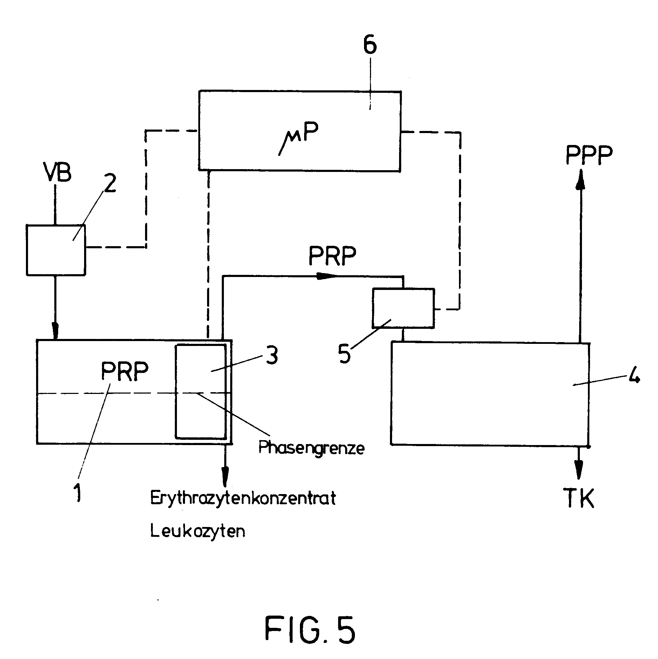

In Figur 5 ist schematisch eine Blutzentrifuge dargestellt mittels der das Verfahren nach der Erfindung vorzugsweise durchführbar ist. Der besseren Übersicht halber sind nur die wesentlichen Bestandteile dieser Blutzentrifuge dargestellt. Die Blutzentrifuge, auch Blutzellseparator genannt, weist eine erste Trennstufe, eine Separationskammer 1 auf, der eingangsseitig über eine Blutpumpe 2 das zu trennende Blut, das sog. Vollblut VB, zuführbar ist. In dieser Separationskammer 1 wird das Vollblut grob in zwei Phasen getrennt. Die erste Fraktion besteht vorzugsweise aus Blutzellen, insbesondere den Erythrozyten RBC. Die zweite Fraktion besteht vorzugsweise aus dem thrombozytenreichen Blutplasma PRP. Weitere Einzelheiten zu den einzelnen Fraktionen und deren Veränderung abhängig von der Separationsdauer können den Figuren 1 und 2 in Verbindung mit den Tabellen 1 und 2 entnommen werden. Der Separationskammer 1 ist eine Einrichtung 3 zur Erfassung der Phasengrenze zwischen den beiden Fraktionen, der sogenannte Phasendetektor, zugeordnet.

Die Blutzentrifuge weist ferner eine zweite Trennstufe, eine Sammelkammer 4 auf, der über eine Plasmapumpe 5 die thrombozytenreiche Blutplasmafraktion PRP zuführbar ist. In dieser Sammelkammer wird die PRP-Fraktion zwecks Gewinnung des Thrombozytenkonzentrates TK weiter separiert.

Die Blutzentrifuge besitzt ferner eine Programmsteuerung 6, die üblicherweise durch einen Mikroprozessor gebildet wird. Diese Steuerung ist mit allen notwendigen Sensoren und Stellgliedern der Blutzentrifuge verbunden und steuert sämtliche Betriebszustände. Die Verbindung zwischen dem Phasendetektor 3 und der Programmsteuerung 6 und von dieser zur Plasmapumpe 5 ist in der Regel so getroffen, daß die Flußrate der Plasmapumpe verändert wird, sobald eine Lageverschiebung der Phasengrenze eintritt oder eintreten soll.

Die Einstellung der Phasengrenze zwischen den beiden Fraktionen in der Separationskammer erfolgt durch Vorgabe des Verhältnisses zwischen den Flußraten der Plasma- und Blutpumpe.

Befindet sich die Blutzentrifuge im Grundzustand (steadystate) an der unteren Trenngrenze, so fördern Blut- und Plasmapumpe in einem vorgegebenen ersten Verhältnis (z.B. 50/20). Soll die Phasengrenze hochgefahren werden, muß dieses Verhältnis zugunsten der Plasmaförderrate verändert werden. Die Programmsteuerung übernimmt dabei die notwendigen Einstellungen. Üblicherweise wird hierzu die Plasmapumpe hochgefahren. Sobald die obere vorbestimmte Trenngrenze erreicht ist, fördern die Pumpen im alten Verhältnis (z.B. 50/20).

Beim Herunterfahren erfolgt eine umgekehrt proportionale Steuerung. Die Plasmapumpe wird auf eine niedrigere Pumpgeschwindigkeit eingestellt, pumpt daher weniger ab, bis die untere Trenngrenze erreicht ist.

Erfindungsgemäß weist die Programmsteuerung einen Steuerabschnitt für die Plasmapumpe 5 auf, der so ausgebildet ist, daß in der Separationskammer 1 während des Trennvorganges die Phasengrenze zwischen beiden Fraktionen alternierend zwischen mindestens zwei Stellungen verschiebbar ist. Der Steuerabschnitt weist dabei einen ersten Zeitabschnitt für die Einstellung einer niedrigeren bis hohen Phasengrenze und einen zweiten Abschnitt für die Einstellung einer extrem hohen Phasengrenze auf. Dabei ist der erste Zeitabschnitt, der Sammelabschnitt, länger als der zweite Zeitabschnitt, der Transferabschnitt.

Auch ist eine Verfahrenssteuerung der Blutzentrifuge orientiert an der angehäuften Thrombozytenmenge der jeweiligen Einzelspender möglich; damit können individuelle Unterschiede im Spenderblut, wie Blutplättchengröße, Plättchenkonzentration usw. berücksichtigt werden.

## Patentansprüche

1. Verfahren zum Trennen von Blut in seine Bestandteile durch Dichtezentrifugation, bei dem in einer ersten Trennstufe das Blut grob in mindestens zwei Phasen separiert wird, von denen die erste Fraktion vorwiegend aus Blutzellen und die zweite Fraktion vorwiegend aus thrombozytenreichem Blutplasma besteht, und bei dem in einer zweiten Trennstufe eine der Fraktionen zwecks Gewinnung eines Konzentrates eines Blutbestandteiles weiter separiert wird, dadurch gekennzeichnet, daß in der ersten Trennstufe während des Trennvorgangs die Phasengrenze zwischen beiden Fraktionen alternierend zwischen mindestens zwei Stellungen verschoben wird.

2. Verfahren nach Anspruch 1, dadurch gekennzeichnet, daß für einen ersten Zeitabschnitt eine niedrige bis hohe Phasengrenze eingestellt wird, und daß für einen zweiten Zeitabschnitt eine extrem hohe Phasengrenze eingestellt wird.

3. Verfahren nach Anspruch 2, dadurch gekennzeichnet, daß der erste, der Sammelzeitabschnitt, länger ist als der zweite, der Transferabschnitt.

4. Verfahren nach Anspruch 3, dadurch gekennzeichnet, daß die Ermittlung der Zeitabschnitte und/oder der Phasengrenze-Positionen empirisch erfolgt.

5. Verfahren nach Anspruch 3, dadurch gekennzeichnet, daß die Bestimmung der Zeitabschnitte und/oder der Phasengrenze-Positionen selbsttätig in Abhängigkeit von der angehäuften Menge des zu gewinnenden Blutbestandteils erfolgt.

6. Verfahren nach Anspruch 3, 4 oder 5, dadurch gekennzeichnet, daß für die Dauer der Separation aufeinanderfolgend mindestens 2 Sammelzeitabschnitte und mindestens Transferabschnitte vorgesehen sind.

7. Vorrichtung zur Trennung von Blut in seine Bestandteile durch Dichtezentrifugation, insbesondere zur Durchführung der Verfahren nach Anspruch 1 oder einem der folgenden, mit einer ersten Trennstufe, der Separationskammer (1), der über eine Blutpumpe (2) das zu trennende Blut zuführbar ist, in der das Blut grob in mindestens zwei Phasen separiert wird, von denen die erste Fraktion vorwiegend aus Blutzellen und die zweite Fraktion vorwiegend aus thrombozytenreichem Blutplasma besteht, und mit einer zweiten Trennstufe, der Sammelkammer (4), der über eine Plasmapumpe (5) die thrombozytenreiche Blutplasma-Fraktion zuführbar ist, und in der diese Fraktion zwecks Gewinnung eines Konzentrates eines Blutbestandteils weiter separierbar ist, und mit einer Programmsteuerung zum Betreib der Vorrichtung, einschließlich der Einstellung der Pumpgeschwindigkeit der Plasmapumpe (5), dadurch gekennzeichnet, daß die Programmsteuerung (6) einen Steuerabschnitt für die Plasmapumpe (5) aufweist, der so ausgebildet ist, daß in der Separationskammer (1) während des Trennvorganges die Phasengrenze zwischen den beiden Fraktionen alternierend zwischen mindestens zwei Stellungen verschiebbar ist.

8. Vorrichtung nach Anspruch 7, dadurch gekennzeichnet, daß der Steuerabschnitt einen ersten Zeitabschnitt für die Einstellung einer niedrigen bis hohen Phasengrenze und einen zweiten Zeitabschnitt für die Einstellung einer extrem hohen Phasengrenze aufweist.

9. Vorrichtung nach Anspruch 8, dadurch gekennzeichnet, daß der erste, der Sammelzeitabschnitt, länger ist als der zweite, der Transferabschnitt.

## Claims

1. A process for separation of blood into its components by density centrifugation, comprising:
coarsely separating the blood in a first separation stage into at least two phases, of which a first fraction consists primarily of blood cells and the second fraction consists primarily of thrombocyte-rich blood plasma, a phase boundary between the two fractions being shifted alternatingly between at least two positions; and
further separating one of said fractions in a second separation stage to obtain a concentrate of one blood component.

2. The process according to Claim 1, characterized in that for a first time interval a low to high phase boundary is set, and that for a second time interval an extremely high phase boundary is set.

3. The process according to Claim 2, characterized in that the first, collection interval, is longer than the second, transfer interval.

4. The process according to Claim 3, characterized in that the determination of the time intervals and/or the phase boundary positions is performed empirically.

5. The process according to Claim 3, characterized in that the determination of the time intervals and/or the phase boundary positions is performed automatically as a function of the accumulated quantity of the blood component to be obtained.

6. The process according to Claim 3, 4 or 5, characterized in that for the duration of the separation at least 2 collection intervals and at least 2 transfer intervals are provided in succession.

7. A device for separation of blood into its components by density centrifugation, particularly for carrying out the process according to Claim 1 oder any of the following, comprising:
a first separation stage, the separation chamber (1), into which via a blood pump means (2) the blood to be separated is introduced and in which the blood is coarsely separated into at least two phases, of which the first fraction consists primarily of blood cells and the second fraction consists primarily of thrombocyte-rich blood plasma; and
a second separation stage, the collection chamber (4) into which via a plasma pump means (5) the thrombocyte-rich blood plasma fraction is delivered and in which this fraction may be further separated for the purpose of obtaining a concentrate of one blood components; and
program controller means for operation of the device including adjustment of the pump speed of the plasma pumpe (5), characterized in that the program controller means (6) includes a control section for the plasma pump (5), which is designed such that in the separation chamber (1) during the separation process the phase boundary between the two fractions can be shifted alternatingly between at least two positions.

8. The device according to Claim 7, characterized in that the control section has a first time interval for setting a low to high phase boundary and a second time interval for setting an extremely high phase boundary.

9. The device according to Claim 8, characterized in that the first, collection interval is longer than the second, transfer interval.

## Revendications

1. Procédé pour séparer le sang en ses constituants par centrifugation en gradients de densité, selon lequel, lors d'une première étape de séparation, le sang est séparé grossièrement en au moins deux phases, parmi lesquelles la première fraction est constituée de façon prépondérante par des cellules du sang et la seconde fraction est constituée essentiellement par du plasma sanguin riche en thrombocytes, et selon lequel lors d'une seconde étape de séparation, l'une des fractions est à nouveau séparée en vue de l'obtention d'un concentré d'un constituant du sang, caractérisé en ce que lors de la première étape de séparation, pendant l'opération de séparation la limite de phase entre les deux fractions est décalée alternativement entre au moins deux positions.

2. Procédé selon la revendication 1, caractérisé en ce qu'une limite de phase d'une valeur faible jusqu'à une valeur élevée est réglée pendant un premier intervalle de temps et qu'une limite de phase extrêmement élevée est réglée pendant un second intervalle de temps.

3. Procédé selon la revendication 2, caractérisé en ce que le premier intervalle de temps, c'est-à-dire l'intervalle de temps de collecte, est plus long que le second intervalle de temps, à savoir l'intervalle de temps de transfert.

4. Procédé selon la revendication 3, caractérisé en ce que la détermination des intervalles de temps et/ou des positions des limites de phase s'effectue d'une manière empirique.

5. Procédé selon la revendication 3, caractérisé en ce que la détermination des intervalles de temps et/ou des positions des limites de phase s'effectue automatiquement en fonction de la quantité accumulée du constituant du sang devant être obtenu.

6. Procédé selon la revendication 3, 4 ou 5, caractérisé en ce que successivement au moins 2 intervalles de temps de collecte et au moins des intervalles de temps de transfert sont prévus successivement pendant la durée de la séparation.

7. Dispositif pour séparer le sang en ses constituants par centrifugation en gradients de densité, notamment pour la mise en oeuvre du procédé selon la revendication 1 ou l'une des suivantes, comportant un premier étage de séparation, à savoir la chambre de séparation (1), auquel le sang séparé peut être envoyé par l'intermédiaire d'une pompe à sang (2) et dans laquelle le sang est séparé de façon approximative en au moins deux phases, parmi lesquelles la première fraction est constituée de façon prépondérante par des cellules du sang et la seconde fraction est constituée de façon prépondérante par du plasma sanguin riche en thrombocytes, et comportant un second étage de séparation, à savoir la chambre de collecte (4), auquel la fraction du plasma sanguin riche en thrombocytes peut être envohée par l'intermédiaire d'une pompe à plasma (5), et dans laquelle cette fraction peut être séparée de façon supplémentaire en vue de l'obtention d'un concentré d'un constituant du sang, et comportant une unité de commande programmée pour faire fonctionner le dispositif, y compris le réglage de la vitesse de la pompe à plasma (5), caractérisé en ce que l'unité de commande programmée (6) comporte une section de commande pour la pompe à plasma (5), qui est agencée de telle sorte que dans la chambre de séparation (1), pendant le processus de séparation, la limite de phase entre les deux fractions est déplaçable d'une manière alternée entre au moins deux positions.

8. Dispositif selon la revendication 7, caractérisé en ce que la section de commande possède un premier intervalle de temps pour le réglage d'une limite de phase allant d'un niveau faible jusqu'à un niveau élevé, et un second intervalle de temps pour le réglage d'une limite de phase extrêmement élevée.

9. Dispositif selon la revendication 8, caractérisé en ce que le premier intervalle de temps, à savoir l'intervalle de temps de collecte, est plus long que le second intervalle de temps, à savoir l'intervalle de temps de transfert.
